Europäisches Patentamt
European Patent Office
Office européen des brevets

(19)

(11) Publication number: **0 415 585 A1**

(12) # EUROPEAN PATENT APPLICATION

(21) Application number: 90308806.0

(22) Date of filing: 09.08.90

(51) Int. Cl.5: C07C 229/60, C07C 255/58

(30) Priority: 31.08.89 GB 8919678

(43) Date of publication of application: 06.03.91 Bulletin 91/10

(84) Designated Contracting States: AT BE CH DE ES FR GB IT LI LU NL SE

(71) Applicant: IMPERIAL CHEMICAL INDUSTRIES PLC
Imperial Chemical House, Millbank
London SW1P 3JF(GB)

(72) Inventor: Moilliet, John Stewart
4 Hunters Green, Holcombe Brook
Bury, Lancashire BL0 9TH(GB)
Inventor: Jones, Ian Kevin
444 Kingsway
Burnage, Manchester M19 1QJ(GB)

(74) Representative: Pugsley, Roger Graham et al
IMPERIAL CHEMICAL INDUSTRIES PLC Legal Department: Patents PO Box 6 Bessemer Road
Welwyn Garden City Herts, AL7 1HD(GB)

(54) **Fluorobenzene derivatives.**

(57) A compound of Formula (2):

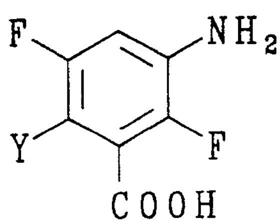

Formula (2)

wherein: Y is -F or -Cl;
and a process for its preparation from a compound of Formula (3):

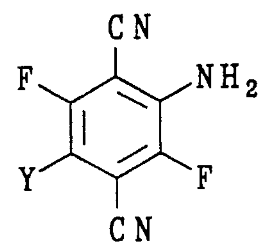

Formula (3)

wherein: Y is -F or -Cl; with a process for the preparation of a compound of Formula (3) from a compound of Formula (4):

EP 0 415 585 A1

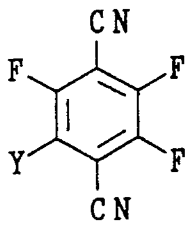

Formula (4)

wherein: Y is -F or -Cl;
and a process for the conversion of a compound of Formula (2) into a compound of Formula (1):

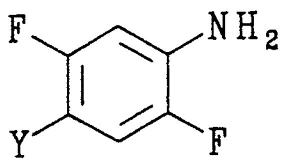

Formula (1)

are provided.

Compounds of Formula (1) are useful as intermediates in the manufacture of agrochemicals, pharmaceuticals and dyestuffs.

## FLUOROBENZENE DERIVATIVES

This invention relates to processes for the preparation of fluoroaromatics and to certain novel compounds.

The preparation of certain trihaloanilines is known from GB 1131501 which describes the preparation of 2,4,5-trifluoroaniline from 1,2,4,5-tetrafluorobenzene by reaction with gaseous ammonia in methanol but a poor yield of the desired product is obtained. This is because mixed products which include the N-methyl and N,N-dimethyl derivatives are formed during the reaction. An alternative route published in J.Gen.Chem. U.S.S.R. 31 1229 involves the preparation of 1,2,4-trifluorobenzene by multiple Schiemann reactions followed by nitration before reducing the nitro derivative to 2,4,5-trifluoroaniline.

It is an object of the present invention to provide a route to 2,4,5-trihaloanilines which gives a high yield and is generally convenient to operate.

According to a first-feature of the present invention there is provided a compound of the Formula (2):

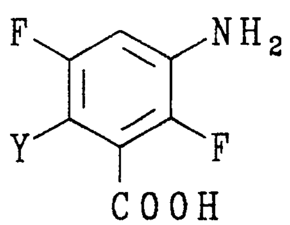

Formula (2)

wherein: Y is -F or -Cl.

The compound of the Formula (2) may be prepared by acid hydrolysis of a compound of the Formula (3):-

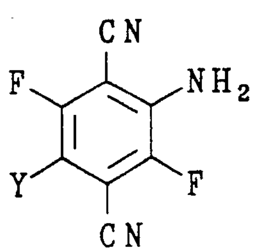

Formula (3)

wherein: Y is -F or -Cl.

The compound of the Formula (3) may be prepared by a process which comprises reacting a compound of the Formula (4):-

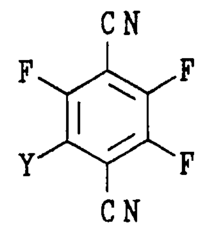

Formula (4)

wherein: Y is -F or -Cl;
with gaseous ammonia.

The compound of Formula (4) in which Y is -Cl may be prepared from 2,3,5,6-tetrachloro-1,4-benzodinitrile by partial fluorination in a halogen exchange reaction with potassium fluoride. The desired isomer being separated and purified by vacuum sublimation.

According to another feature of the present invention there is provided a process for the preparation of a compound of the Formula (1):

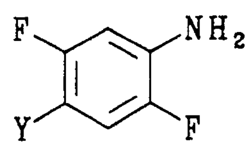

Formula (1)

which comprises decarboxylating a compound of the Formula (2):

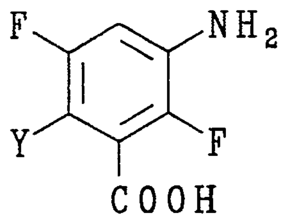

Formula (2)

wherein: Y is -F or -Cl.

The compounds of Formulae (2) and (3) are novel and form a further feature of the present invention. Specific examples of compounds of Formulae (1), (2) and (3) are those in which Y is -F and in which Y is -Cl.

The decarboxylation of the compound of Formula (2) is preferably effected by heating in an inert neutral or basic liquid. Examples of suitable liquids include dimethylacetamide, sulpholane and diethylene glycol dimethyl ether. The process is preferably performed at a temperature from 100°C to 165°C, especially 130°C to 160°C and more especially 150°C. Reaction is preferably continued until all the starting material is consumed which can take up to 12 hours, although decarboxylation is generally substantially complete within 2 hours.

The hydrolysis of compounds of Formula (3) is preferably effected in an acid medium. The compound of Formula (3) is preferably added to a suitable acid solution at a temperature from 50°C to 180°C, more preferably at 100° to 170 °C especially at 130° to 165°C. An example of a suitable acid solution is 77% sulphuric acid. The reaction is preferably continued until substantially complete which can take up to 7 hours.

In the preparation of compounds of Formula (3) by reaction of a compound of Formula (4) with ammonia the compound of Formula (4) is preferably stirred in an inert solvent at a temperature from 40°C to 80°C, more preferably 50°C to 70°C and especially 60°C and ammonia gas is passed over the surface of the reaction slurry until all the starting material is consumed. An example of a suitable solvent is 1,2-dimethoxyethane.

Compounds of Formulae (1), (2), (3) and (4) are useful as intermediates in the preparation of agrochemicals, pharmaceuticals and dyestuffs.

The invention is further illustrated by the following examples in which all parts and percentages are by weight unless otherwise indicated.

Example 1

Preparation of 2,4,5-trifluoroaniline

300 parts of dimethyl acetamide was stirred at 5°C and 148 parts of 3-amino-2,5,6-trifluorobenzoic acid was added. The temperature was raised to 150°C, when $CO_2$ was evolved, and the temperature maintained at 150°C for 1½ hours. The reaction mixture was distilled up to 190°C and the distillates drowned into 1500 parts of water with stirring. The white solid was filtered off, washed with water and dried to give 46 parts of product.

The aqueous dimethyl acetamide was extracted with 2 x 500 parts of $CH_2Cl_2$ which in turn was washed with 3 x 250 parts of water. The $CH_2Cl_2$ solution as dried and evaporated to give 21 parts of solid.

A total of 67 parts (60%) of 2,4,5-trifluoroaniline was obtained.

Comparative Example 1

Preparation of 2,4,5-trifluoroaniline

The preparative route of GS 1131501 was used to prepare 2,4,5-trifluoroaniline as a comparison with the route of the present invention. Ammonia gas (51 parts) was dissolved in methanol (354 parts) and charged to a 1 litre enamel lined autoclave. 1,2,4,5-tetrafluorobenzene (30 parts) was added, the autoclave was sealed and heated to 250°C for 18 hours. The volatile material comprising methanol, ammonia and starting material was removed by distillation to leave a black oil. The black oil was a mixture comprising 2,4,5-trifluoroaniline (7.8 parts 25.0%), N-methyl2,4,5-trifluoroaniline (1.3 parts 4.2%) and N,N-dimethyl-2,4,5-trifluoroaniline (3.9 parts 12.6%). Example 2

Preparation of 3-amino-2,5,6-trifluorobenzoic acid

1,527 parts of 77% sulphuric acid was stirred and heated at 130°C while 305 parts of 2-amino-3,5,6-trifluoro-1,4-benzodinitrile was added in small portions over 1- hours. The temperature of the reaction mixture was raised to 165°C over 4 hours and held for a further 2 hours until gas chromatographic (GC) analysis showed it was complete. After cooling to 70°C the solution was poured onto 1500 parts of ice and water and the pH of the solution formed was adjusted to 2.5 by the addition of a 47% sodium hydroxide solution. During this addition the temperature was maintained at less than 60°C by applying external cooling. The resultant slurry was cooled to 20°C and filtered.

The filter cake was washed with 3 x 500 parts

of ethyl acetate, and this solution was washed with water, dried over $MgSO_4$ then evaporated to give 220 parts crude product. The aqueous filtrates were extracted with 3 x 500 parts of ethyl acetate, which after drying and evaporation gave a further 39 parts of product.

The crude product was recrystallised from water to give 205 parts (70%) of 3-amino-2,5,6-trifluorobenzoic acid.

Example 3

Preparation of 2-amino-3,5,6-trifluoro-1,4-benzodinitrile

A mixture of 400 parts of 2,3,5,6-tetrafluoro-1,4-benzodinitrile in 1,400 parts of 1,2-dimethoxyethane was stirred at 60° C while ammonium gas was passed over the surface of the reaction slurry. The reaction, which was exothermic, was followed by GC analysis until there was no starting material left. The reaction mixture was cooled and the liquid poured into 8000 parts of water and stirred for half an hour. The solid was filtered off, washed with 2 x 1000 parts of water and dried to give 359 parts (91%) of 2-amino-3,5,6-trifluoro-1,4-benzodinitrile.

**Claims**

1. A compound of the Formula (2):

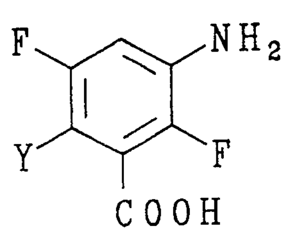

Formula (2)

wherein: Y is -F or -Cl.

2. A process for the preparation of a compound according to Claim 1 which comprises hydrolysing a compound of the Formula (3):

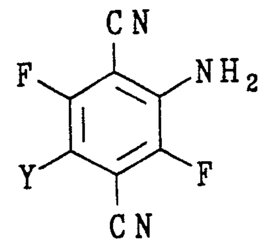

Formula (3)

in an acid medium
wherein: Y is -F or -Cl.

3. A process according to Claim 2 wherein the hydrolysis is effected by treating the compound of Formula (2) with an acid at a temperature from 50 to 180°.C.

4. A compound of the Formula (3):

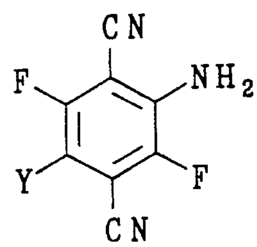

Formula (3)

wherein: Y is -F or -Cl.

5. A process for the preparation of a compound according to Claim 4 which comprises reacting a compound of the Formula (4):

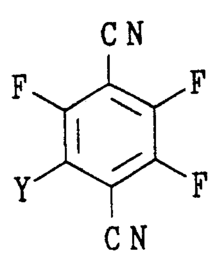

Formula (4)

wherein: Y is -F or -Cl, with gaseous ammonia.

6. A process according to Claim 5 wherein the ammonia is passed over the surface of a mixture of the compound of Formula (3) and an inert liquid at

a temperature from 40 to 80 ° C.

7. A process for the preparation of a compound of the Formula (1):

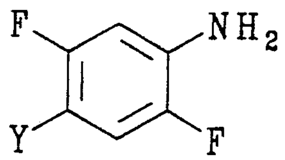

Formula (1)

comprising decarboxylating a compound of the Formula (2):

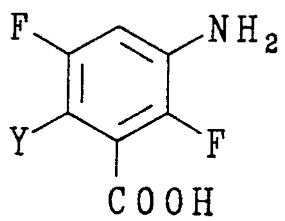

Formula (2)

wherein: Y is -F or -Cl.

European Patent Office

# EUROPEAN SEARCH REPORT

Application Number

EP 90 30 8806

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | GB-A-2 165 239 (S.D.S. BIOTECH. K.K.) * pages 1-6; page 8, lines 39-51; claim 1 * | 4-6 | C 07 C 229/60<br>C 07 C 255/58 |
| A | EP-A-0 281 266 (NIPPON SHOKUBAI KAGAKU KOGYO) * the whole document * | 1-3 | |
| A | EP-A-0 053 247 (BASF AG) * the whole document * | 2,5 | |
| A | GB-A- 991 537 (FARBENFABRIKEN BAYER AG) * claim 1 * | 1 | |
| A | US-A-4 556 426 (J.J. CHESNEY et al.) * column 3; claim 1 * | 5 | |
| | | | **TECHNICAL FIELDS SEARCHED (Int. Cl.5)** |
| | | | C 07 C 229/00<br>C 07 C 255/00 |

The present search report has been drawn up for all claims

| Place of search | Date of completion of the search | Examiner |
|---|---|---|
| BERLIN | 24-09-1990 | RUFET J.M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)